# FASCICULE DE BREVET EUROPEEN

(11) **EP 1 827 684 B1**
(45) Date de publication et mention de la délivrance du brevet: **20.02.2019**
(21) Numéro de dépôt: 05818277.5
(22) Date de dépôt: 17.11.2005
(51) Int. Cl.: B01J 23/75, C07C 1/04, B01J 21/12

(54) **CATALYSEUR A BASE DE COBALT POUR LA SYNTHESE FISCHER-TROPSCH**
AUF COBALT BASIERENDER KATALYSATOR FÜR DIE FISCHER-TROPSCH-SYNTHESE
COBALT-BASED CATALYST FOR FISCHER-TROPSCH SYNTHESIS

(30) Priorité: 17.12.2004 FR 0413545; 26.10.2005 FR 0511014
(43) Date de publication de la demande: 05.09.2007
(73) Titulaire: IFP Energies nouvelles, 92500 Rueil-Malmaison (FR); ENI S.p.A., Roma (IT)
(72) Inventeur: DIEHL, Fabrice, F-69007 Lyon (FR); HUGUES, François, F-69390 Vernaison (FR); MARION, Marie-Claire, F-69390 Vernaison (FR); UZIO, Denis, 69220 Belleville (FR)
(74) Mandataire: IFP Energies nouvelles
(86) Numéro de dépôt international: PCT/FR2005/002863
(87) Numéro de publication internationale: WO 2006/067285

(56) Documents cités:
- EP-A- 1 233 011
- WO-A-99/42214
- WO-A-02/068117
- US-A- 3 909 455
- US-A- 4 497 903
- US-A- 4 801 620

## Description

### Domaine de l'invention

Le domaine de l'invention est celui des procédés de synthèse Fischer Tropsch qui permettent d'obtenir une large gamme de coupes hydrocarbonées à partir du mélange CO , H₂, (monoxyde de carbone et hydrogène) communément appelé gaz de synthèse.

Les catalyseurs utilisés dans ce type de réactions sont le plus souvent des catalyseurs supportés à base d'alumine ou de silice alumine, la phase active étant constitué de fer (Fe) ou de cobalt (Co).

La présente invention décrit un nouveau type de catalyseur qui, par sa structure, va permettre une meilleure résistance à l'attrition, et donc diminuer les problèmes liés à la séparation des effluents pouvant contenir une certaine proportion de particules fines de catalyseur, ainsi que réduire le risque de contamination des catalyseurs utilisés dans les unités avales.

Le gaz de synthèse est un mélange de monoxyde de carbone et d'hydrogène présentant des ratios molaires H₂/CO pouvant varier dans un rapport de 0,5 à 4 en fonction du procédé par lequel il a été obtenu.
- à partir du procédé de vaporeformage d'hydrocarbures ou d'alcool, le rapport H₂/CO du gaz de synthèse est généralement voisin de 3,
- à partir d'un procédé d'oxydation partielle le rapport H₂/CO du gaz de synthèse est plutôt voisin de 1,5 à 2,
- à partir d'un procédé de reformage thermique le rapport H₂/CO du gaz de synthèse est généralement voisin de 2,5,
- à partir d'un procédé de gazéification et de reformage du CO_{2,} le rapport H₂/CO du gaz de synthèse est généralement voisin de 1.

Le gaz de synthèse est notamment utilisé dans la synthèse Fischer-Tropsch pour la production d'hydrocarbures supérieurs (C5+), essentiellement linéaires et saturés, contenant au moins 50% pds d'hydrocarbures C5+ par rapport à l'ensemble des produits formés.

L'équation stoechiométrique simplifiée de la synthèse Fischer-Tropsch s'écrit:

n CO + (2n+1) H₂ -> CₙH₂ₙ₊₂ + n H₂O

Cette réaction est généralement réalisée à moyenne ou haute température et sous pression.

Elle est connue de l'homme du métier pour être catalysée par les métaux du groupe VIII de la classification tels que le cobalt, le nickel, le ruthénium ou encore le fer.

La réaction de synthèse Fischer-Tropsch peut être réalisée dans différents types de réacteurs (lit fixe, mobile, ou triphasique (gaz, liquide, solide) par exemple de type autoclave parfaitement agité, ou colonne à bulles), et les produits de la réaction présentent notamment la caractéristique d'être exempts de composés soufrés, azotés ou de type aromatique.

Les produits de la réaction de synthèse Fischer-Tropsch peuvent être valorisés par des séparations ou des transformations chimiques ultérieures. Ainsi, la coupe C5-C15 peut être distillée, et les hydrocarbures correspondant utilisés comme solvants. Un hydrotraitement peut permettre d'augmenter la pureté de ces produits en éliminant les traces d'oléfines.

Les procédés de transformation tel que l'hydrocraquage, le déparaffinage, ou l'hydroisomérisation des coupes plus lourdes (C16+) permet de produire différents types de carburants dans la gamme des distillats moyens: gazole (coupe 180-370°C), kérosène (coupe 140- 300°C).

On appelle "slurry" dans le contexte de l'invention une mise en oeuvre du catalyseur caractérisée par le fait que celui ci est divisé à l'état de poudre très fines, typiquement de l'ordre de quelques dizaines de microns, cette poudre formant une suspension avec le milieu réactionnel. On conservera dans la suite du texte la terminologie "slurry" bien connue de l'homme du métier, pour désigner le type de mise en oeuvre précédemment défini.

Lors de sa mise en oeuvre dans les procédés de synthèse Fischer-Tropsch, et notamment dans les procédés de type "slurry", au sens précédemment défini, le catalyseur est soumis à des conditions particulièrement sévères en terme d'attrition mécanique et chimique.

En effet, les vitesses linéaires très élevées rencontrées dans les procédés "slurry" génèrent des chocs inter particulaires ou contre les parois des équipements, chocs qui peuvent entraîner la formation de fines.

On entend par fines des particules de diamètre inférieur à 10 microns, éventuellement inférieur à 1 micron, auquel cas on parle de particules submicroniques. Ces particules fines sont particulièrement difficiles à séparer des effluents de la réaction, et peuvent contaminer les catalyseurs utilisés dans les procédés situés en aval. Additionnellement à ces efforts mécanique, le solide travaille en conditions hydrothermales poussées, c'est à dire sous des pressions partielles en vapeur d'eau (l'eau étant un coproduit fatal de la réaction) variant depuis quelques dizièmes de méga Pascal à des valeurs au-delà de 1MPa en fonction du taux de conversion du monoxyde de carbone.

Dans ces conditions, l'utilisation de supports à base d'alumine ou de silice peut présenter des risques liés au phénomène de réhydratation inhérent aux conditions de la réaction essentiellement définies par le couple température/pression partielle d'eau. Ce phénomène de réhydratation, décrit notamment dans les travaux de J.P. Franck, Chem Communications n°1071984, altère chimiquement le support et conduit à la formation de composés hydratés de type boehmite ou kaolinite en fonction du matériau de départ.

Dans le cas d'utilisation en réacteur triphasique ("slurry"), cette altération chimique, alliée aux conditions hydrodynamiques sévères décrites précédemment, conduit à une attrition marquée.

Un des moyens parmi les plus efficaces pour réduire l'ampleur de ce phénomène de réhydratation est de modifier la composition du support, soit en limitant la dite modification à la surface du support, soit en modifiant également la composition et la structure volumique de ce support.

### Examen de l'art antérieur

De nombreux travaux ont donc été effectués dans le but de stabiliser le support vis à vis de ces processus de réhydratation, et/ou de redissolution du support.
- Ainsi, il est enseigné dans le brevet WO 99/42214 que l'introduction d'éléments choisis parmi les éléments Si, Zr, Cu, Zn, Mn, Ba, Co, Ni, La permet de limiter sensiblement le processus de réhydratation/dissolution du support en milieu aqueux acide ou neutre.

Une manière préférée de réaliser la modification du support consiste à greffer à la surface du dit support des composés organiques du Si de type TEOS: (TétraEthylOrthoSilicate) ou TMOS (TriMéthOxySilane).
- Dans le brevet WO 02/07883, il est enseigné que la modification d'un support de catalyseur de synthèse Fischer-Tropsch par imprégnation d'un composé organométallique de formule Me(OR)ₓ avec x variant de 1 à 5, Me étant un métal choisi parmi les éléments suivants Si, Zr, Cu, Zn, Mn, Ba, Co, Ni, Na, K, Ca, Sn, Cr, Fe, Li, Tl, Mg, Sr, Ga, Sb, V, Hf, Th, Ce, Ge, U, Nb, Ta, W, et R désignant un groupement alkyle ou acyle, permet de limiter la formation de phases cristallisées responsables de la perte d'activité du catalyseur dans les conditions de fonctionnement de la synthèse Fischer-Tropsch.

Le composé organométallique peut éventuellement être décomposé par calcination après dépôt de la phase active par imprégnation. L'inconvénient majeur de ce mode de préparation est l'utilisation d'un solvant organique destiné à solubiliser le composé organométallique avant son dépôt par imprégnation.
- Il est enseigné dans les brevets US 5 169 821 et US 5 397 806 que l'introduction de silicium, zirconium ou de tantale dans un catalyseur à base de cobalt supporté sur oxyde de titane de type anatase permettrait d'obtenir un effet stabilisant vis à vis d'un traitement régénératif à haute température.
- La stabilisation mécanique vis à vis des conditions d'attrition sévères dans une mise en oeuvre de type "slurry" peut également être obtenue par ajout de silice (SiO₂) et d'alumine (Al₂O₃) à une phase initiale d'oxyde de titane (TiO₂) comme présenté dans le brevet WO99/39825.
- L'utilisation des phases de structure spinelle MAl₂O₄ en catalyse (vaporeformage d'hydrocarbure, oxydation partielle, désalkylation à l'eau) est généralement motivée par les propriétés de surface particulières de ces solides, ainsi que leur excellente stabilité texturale et mécaniques dans le domaine des hautes températures requises pour ces applications (par exemple dans le reformage du méthane pour la production de gaz de synthèse tel que décrit dans les travaux de Xu Z, Li Y, Zhang J, Chang L, Zhou R, Duan Z in Applied Catal A General, (2001), 213(1), 65-71).

Les spinelles simples (AB₂O₄) ou mixtes (AₓA'_{1-X}B₂O₄) sont synthétisés en portant à haute température des mélanges d'oxydes métalliques obtenus par coprécipitation de précurseurs métalliques.
- En synthèse Fischer-Tropsch, l'utilisation de support de type spinelle simple a fait l'objet des travaux de Kondo S, Muraki H, Fujitani Y in Sekiyu Gakkaishi (1984) 27 (6), 556-563. L'utilisation de MgAl₂O₄ permet d'obtenir dans les conditions de tests (lit fixe) des catalyseurs plus actifs que sur différentes alumines de transition (γ, δ, α).

La présence de silicium, notamment sous forme de silice-alumine dans le support, n'est cependant pas mentionnée dans les travaux de ces auteurs.

### Description sommaire de l'invention

L'invention concerne donc un catalyseur pour effectuer la synthèse d'hydrocarbures à partir d'un mélange comprenant du monoxyde de carbone et de l'hydrogène (synthèse dite Fischer Tropsch), le dit catalyseur comprenant:
- un support constitué d'une structure spinelle simple de type MAl₂O₄/Al₂O₃.SiO₂ dans lequel M est le cobalt, et Al₂O₃.SiO₂ désignant la formule chimique d'une silice alumine, ledit support comprenant au moins 10 % poids de structure spinelle, ledit support étant un support calciné sous une atmosphère au moins partiellement oxydante, à une température comprise entre 850°C et 900°C;
- une phase active déposée sur le dit support, qui contient un ou plusieurs métaux du groupe VIII, choisi parmi le cobalt (Co), le nickel (Ni), le ruthénium (Ru) ou le fer (Fe), de préférence le cobalt ou le fer et de manière très préférée le cobalt.

Dans le cas où la phase active est constituée de Co, Ni ou Fe, les teneurs en métaux Co, Ni et Fe sont généralement choisies entre 1 et 60 % poids, préférentiellement entre 5 et 30% poids, et dans le cas où la phase active est du ruthénium, la teneur en ruthénium est généralement comprise entre 0,01 et 10% poids, préférentiellement entre 0,05 et 5% poids

Le support du catalyseur selon l'invention est calciné sous une atmosphère au moins partiellement oxydante, à une température comprise entre 850°C et 900°C.

La silice alumine intervenant dans le support du catalyseur selon l'invention contient généralement entre 1 et 30% poids de silice par rapport au produit anhydre.

Le métal du groupe VIII intervenant dans la phase active du catalyseur selon l'invention pourra être de manière préférée le Cobalt.

La phase active du catalyseur selon l'invention peut également contenir au moins un élément additionnel choisi dans le groupe constitué par le ruthénium, le molybdène, le tantale, le platine, le palladium et le rhénium, de préférence le ruthénium ou le rhénium.

### Description détaillée de l'invention

L'invention porte donc sur un catalyseur pour la synthèse Fischer Tropsch dont le support présente une structure spinelle simple, ainsi que sur un mode de fabrication de ce catalyseur.

Il a été montré que l'utilisation de spinelle simple de type MAl₂O₄/Al₂O₃ SiO₂ permettait d'augmenter considérablement la résistance hydrothermale des supports tout en conservant un niveau de performances catalytiques élevé en terme d'activité, de stabilité et de sélectivité dans les conditions de la synthèse Fischer-Tropsch. Ces catalyseurs selon l'invention permettent donc d'obtenir des performances catalytiques élevées (conversion et sélectivité en hydrocarbures C5+, c'est à dire en hydrocarbures ayant au moins cinq atomes de carbone) tout en limitant les risques de désactivation dans le temps grâce à une résistance mécanique et à une résistance hydrothermale élevées.

Le catalyseur selon la présente invention comprend:
a) un support constitué d'une structure spinelle simple de formule MAl₂O₄/Al₂O₃ SiO₂ dans lequel M est le Co
b) une phase active déposée sur le dit support, qui contient un ou plusieurs métaux du groupe VIII, choisis parmi le Cobalt (Co), le Nickel (Ni), le Ruthénium (Ru) ou le Fer (Fe). Dans le cas des métaux Co, Ni et Fe, les teneurs pourront être choisies entre 1 et 60 % poids, préférentiellement entre 5 et 30% poids. Dans le cas du ruthénium, une gamme allant de 0,01 à 10% poids, et préférentiellement de 0,05 à 5% poids sera préférée.

Le support du catalyseur selon l'invention comprend au moins 10 % poids de ladite structure spinelle, et de manière-encore plus préférée au moins 15 % poids.

Le catalyseur est préférentiellement mis en oeuvre en réacteur fluidisé triphasique de type autoclave parfaitement agité, ou en colonne à bulle. L'utilisation en lit fixe peut également être envisagée.

Le support de départ est préférentiellement une silice-alumine dont la partie alumine est une alumine de transition constituée d'au moins une phase de structure cristallographique δ, γ, θ ou encore α.

La morphologie du support pourra être de type bille, ou extrudé, (par exemple de forme trilobe), ou pastilles pour des mises en oeuvre en réacteur de type lit fixe, ou en poudre de granulométrie variable pour une mise en oeuvre en réacteur de type "slurry". L'invention n'est pas liée à une mise en oeuvre particulière du catalyseur, mais est au contraire compatible avec tous les types de mise en oeuvre usuels dans la synthèse Fischer-Tropsch, c'est à dire la mise en oeuvre en réacteur de type autoclave parfaitement agité, ou en lit bouillonnant, ou bien encore en lit fixe ou mobile.

De ce fait, la taille des grains peut être comprise entre quelques microns et quelques centaines de microns. Typiquement pour une mise en oeuvre en réacteur triphasique "slurry", la taille des particules de catalyseur sera comprise entre 10 microns et 500 microns, de manière préférée entre 10 microns et 300 microns, de manière très préférée entre 20 et 150 microns, et de manière encore plus préférée entre 20 et 120 microns.

Le support pourra contenir en plus de l'alumine, au moins un oxyde choisi dans le groupe constitué par la silice, les silice-alumines, la cérine, la zircone, ou une combinaison quelconque d'au moins deux de ces oxydes.

Le support préféré selon l'invention est une silice alumine de teneur comprise entre 0,5 et 30% masse de silice par rapport au produit anhydre. De préférence cette silice-alumine est homogène à l'échelle du micromètre, et de manière encore plus préférée homogène à l'échelle du nanomètre.

Tout procédé permettant d'obtenir le dit support, modifié par l'addition d'au moins un élément M pour obtenir la structure spinelle simple convient.

Sans que cette liste puisse être considérée comme limitative, il est possible d'imprégner comme ci-après un support alumine préformé ou en poudre avec au moins une solution aqueuse contenant les précurseurs hydrosolubles des éléments choisis, et de procéder à des étapes de lavage, séchage et enfin de calcination.

De même, il est possible de préparer le dit support par coprécipitation d'une solution aqueuse contenant les métaux Al et M, par exemple sous forme de nitrates, par une solution aqueuse de carbonate ou d'hydrogénocarbonate alcalin, suivie d'un lavage, d'un séchage, et enfin d'une calcination.

Il est également possible de préparer le dit support par le procédé sol-gel, ou encore par complexation de la dite solution aqueuse contenant les métaux Al et M par au moins un acide alpha-alcool ajouté à raison de 0,5 à 2 moles d'acide par mole de métaux, suivi d'un séchage sous vide conduisant à l'obtention d'une substance vitreuse homogène, puis d'une calcination.

Le catalyseur ainsi préparé subit de préférence un traitement thermique destiné à transformer le précurseur M et l'alumine en structure de type spinelle (aluminate de M). Ce traitement thermique est généralement effectué sous atmosphère oxydante (air ou O₂) à haute température, entre 850°C et 900°C, ou encore sous air appauvri en oxygène.

A titre d'exemple, il est possible de déposer le métal actif du groupe VIII par la technique d'imprégnation à sec qui consiste à mettre en contact le support poreux avec une solution dont le volume est égal au volume poreux du support à imprégner. Cette solution contient les précurseurs métalliques du ou des métaux du groupe VIII (chlorure, nitrate, acétate...) à la concentration voulue. L'imprégnation dudit métal actif peut être effectuée en une seule ou plusieurs étapes d'imprégnation. Dans le cas de teneurs en métaux relativement élevées, l'imprégnation en deux étapes, voire en trois étapes est préférée. Ces étapes d'imprégnations viennent en supplément des étapes d'addition de l'élément M. Entre chacune des étapes, il est préféré d'effectuer éventuellement au moins une étape supplémentaire de séchage et/ou de calcination et/ou de réduction. Il peut être très avantageux de déposer sur le catalyseur une quantité faible d'au moins un métal choisi parmi les métaux Pt, Pd, Rh, Ru, Re afin de faciliter la réduction du métal actif du groupe VIII.

Préalablement à son utilisation dans le réacteur catalytique, le catalyseur subit un traitement réducteur, par exemple sous hydrogène pur ou dilué, à haute température destiné à activer le catalyseur et à former des particules de métal à l'état zéro valent. Ce traitement pourra être effectué *in situ* (dans le même réacteur que celui où est opéré la réaction de Fischer-Tropsch), ou *ex situ* avant d'être chargé dans le réacteur.

### Exemples

Dans les exemples qui suivent on compare 5 catalyseurs selon l'art antérieur notés A, B, C, D et G avec deux catalyseurs selon l'invention notés E et F, présentant une structure spinelle simple.

La comparaison est effectuée selon un critère de résistance hydrothermale, un critère de résistance mécanique, et selon un critère d'activité chimique.

Il ressort de cette comparaison que les catalyseur E et F selon l'invention présentent le meilleur compromis entre la résistance hydrothermale, la résistance mécanique, et l'activité chimique.

### Exemple 1: Préparation du catalyseur A de formule Co/Al₂O₃ (comparatif):

Un catalyseur A formé de cobalt sur alumine est préparé par imprégnation à sec d'une solution aqueuse de nitrate de cobalt sur une alumine en poudre (granulométrie moyenne = 90 µm) présentant une surface spécifique de 170 m²/gramme.

Après 12 heures de séchage en statique à 120°C, le solide est calciné 2 heures à 450°C sous un flux d'air en réacteur de type lit traversé. Le catalyseur final A contient 9,2% pds de cobalt.

### Exemple 2: Préparation du catalyseur B Co/Al₂ O₃₋ SiO₂ (comparatif):

Un catalyseur B à base de cobalt sur Silice-alumine est préparé par imprégnation à sec selon la même procédure que pour l'obtention du catalyseur A.

Le support utilisé contient 5% poids de SiO₂ et 95% poids d'alumine (Al₂ O₃). Il présente une surface spécifique de 180 m²/g.

Après activation thermique, le catalyseur B contient 10% poids de cobalt.

### Exemple 3: Préparation du catalyseur C de formule Co/COAl₂O₄-Al₂O₃.SiO₂ (comparatif)

Un catalyseur C est préparé par imprégnation à sec d'une solution de nitrate de cobalt suivi d'une calcination à haute température (800°C) pendant 4 heures sous air.

Le support de départ est la silice-alumine utilisée pour le catalyseur B.

Le support ainsi modifié contient 5% pds de Cobalt inclus dans le support sous la forme d'aluminate de cobalt (CoAl₂O₄).

Une nouvelle étape de dépôt de Cobalt par imprégnation à sec est réalisée selon le même protocole que pour le catalyseur A.

Le catalyseur final C contient 15,4% poids de Cobalt, dont 10,4% sont sous forme d'oxyde de cobalt Co₃O₄.

### Exemple 4: Préparation du catalyseur D de formule Co/Al₂O₃- SiO₂ ex TEOS (comparatif) :

Le support du catalyseur A est imprégné à reflux pendant 4 h par une solution alcoolique (éthanol) de Tetra Ethyl Ortho Silicate (TEOS).

Après séchage sous vide et calcination pendant 12 h à 400°C, le support ainsi modifié et contenant 7,4% poids de SiO₂ est imprégné par une solution aqueuse de nitrate de cobalt selon le même protocole que pour l'exemple 1.

Le catalyseur D final contient 10,1% pds de Cobalt.

La préparation du catalyseur D est réalisée à partir de la publication de S. Barrada, E.A. Caricato, P.J. van Berge et J. van de Loosdrecht dans Studies in Surface Science and Catalysis, (2002), 143, pages 55-65.

### Exemple 5: Préparation du catalyseur E de formule Co/CoAl₂O₄-Al₂O₃.SiO₂ (selon l'invention)

Un catalyseur E est préparé selon le mode de préparation du catalyseur C mais la température de calcination haute température après la première étape d'imprégnation est fixée à 850°C au lieu de 800°C. Le catalyseur final E contient 15,4% poids de cobalt dont 10,4% sont sous forme Co₃O₄.

### Exemple 6: Préparation du catalyseur F de formule Co/CoAl₂O₄-Al₂O₃.SiO₂ (selon l'invention)

Un catalyseur F est préparé selon le mode de préparation du catalyseur C mais la température de calcination haute température après la première étape d'imprégnation est fixée à 900°C au lieu de 800°C. Le catalyseur final F contient 15,4% poids de cobalt dont 10,4% sont sous forme Co3O4.

### Exemple 7: Préparation du catalyseur G de formule Co/CoAl₂O₄-Al₂O₃.SiO₂ (comparatif)

Un catalyseur G est préparé selon le mode de préparation du catalyseur C mais la température de calcination haute température après la première étape d'imprégnation est fixée à 950°C au lieu de 800°C. Le catalyseur final G contient 15,4% poids de cobalt dont 10,4% sont sous forme Co₃O₄.

### Exemple 8: Caractérisation de la résistance hydrothermale:

La caractérisation de la résistance hydrothermale est réalisée en mettant en contact 2 grammes de chacun des catalyseurs étudiés avec un mélange eau, heptane, pentane (respectivement 17% / 48% / 35 % poids) à 200°C pendant 300h dans un autoclave en mode statique sous pression autogène.

Après séchage, le produit est finalement analysé par thermogravimétrie (ATG) couplée à un analyseur infra rouge et un spectromètre de masse pour déterminer la nature des produits dégazés.

Une analyse par diffraction des rayons X est également effectuée pour déterminer la quantité massique de produit hydraté, essentiellement de type boehmite.

Les résultats sont rassemblés dans le tableau suivant :

| | % masse boehmite DRX | perte de masse % 200°C < T < 500°C |
|---|---|---|
| A (comparatif) | 81,0 | 32 |
| B (comparatif) | 17,0 | 13 |
| C (comparatif) | 5,0 | 6 |
| D (comparatif) | 1,0 | 3 |
| E (selon l'invention) | 3,5 | 4,5 |
| F (selon l'invention) | 3,0 | 4 |
| G (comparatif) | 3,0 | 4 |

La perte de masse observée entre 200°C et 500°C correspond au départ d'eau et de dioxyde de carbone, comme l'indiquent le détecteur infrarouge et le spectromètre de masse couplés à l'appareil de thermogravimétrie.

### Exemple 9: Caractérisation de la résistance mécanique des catalyseurs

Après 500 heures de test, la résistance mécanique des catalyseurs A à G a été évaluée par mesure de la granulométrie obtenue après séparation des produits de réaction. Le tableau ci dessous donne le pourcentage de particules de catalyseur présentant une taille inférieure à 20 µm, formées lors des tests des quatre catalyseurs. Cette valeur est représentative de la résistance à l'attrition des catalyseurs

| Catalyseur | % particules inférieures à 20 µm |
|---|---|
| A (comparatif) | 15 |
| B (comparatif) | 8 |
| C (comparatif) | 4 |
| D (comparatif) | 3 |
| E (selon l'invention) | 2,7 |
| F (selon l'invention) | 2,4 |
| G (comparatif) | 2,9 |

Les catalyseurs E et F (selon l'invention) possèdent une résistance mécanique très supérieure à celle des catalyseurs A et B et équivalente à celle des catalyseurs C, D et G.

### Exemple 10: Caractérisation des performances catalytiques en autoclave de type slurry.

Les catalyseurs A à G décrits ci-dessus sont testés dans un réacteur triphasique de type "slurry", parfaitement agité, fonctionnant en continu, et opérant avec une concentration de 10% molaire de catalyseur dans le système triphasique.

Le catalyseur est sous forme de poudre de diamètre compris entre 40 et 150 microns. Les conditions de test sont les suivantes:
- Température= 230°C,
- Pression = 2MPa
- vitesse volumique horaire (VVH) = 1000 h⁻¹
- rapport molaire H₂/CO = 2/1

| Catalyseur | Conversion CO (%vol) après 120h | Distribution des produits formés (% poids) | |
|---|---|---|---|
| | | C1 ^{(a)} | C5+ ^{(b)} |
| A (comparatif) | 55 | 9,0 | 79,0 |
| B (comparatif) | 54 | 12,0 | 75,0 |
| C (comparatif) | 55 | 10,0 | 80,0 |
| D (comparatif) | 55 | 14,0 | 72,0 |
| E (selon l'invention) | 56 | 9,3 | 81,0 |
| F (selon l'invention) | 56 | 9,0 | 82,0 |
| G (comparatif) | 54 | 11,0 | 78,0 |

| | | | |
|---|---|---|---|
| ^{(a)} méthane ^{(b)} hydrocarbures ayant au moins cinq atomes de carbones. | | | |

Les résultats montrent que les catalyseurs E et F selon l'invention présentent des sélectivités en hydrocarbures C5+ meilleures que les catalyseurs non conformes à l'invention. Les catalyseurs E et F conduisent également à des sélectivités en méthane significativement inférieures à celles des catalyseurs A, B, C, D et G non conformes à l'invention.

Ils présentent donc le meilleur compromis entre activité chimique, résistance hydrothermale et résistance mécanique à l'attrition.

## Revendications

1. Catalyseur pour effectuer la synthèse d'hydrocarbures à partir d'un mélange comprenant du monoxyde de carbone et de l'hydrogène, comprenant:
- un support constitué d'une structure spinelle simple de type MAl₂O₄/Al₂O₃.SiO₂ dans lequel M est le cobalt, et Al₂O₃.SiO₂ désignant la formule chimique d'une silice alumine, ledit support comprenant au moins 10 % poids de structure spinelle, ledit support étant un support calciné sous une atmosphère au moins partiellement oxydante, à une température comprise entre 850°C et 900°C;
- une phase active déposée sur le dit support, qui contient un ou plusieurs métaux du groupe VIII, choisi parmi le cobalt, le nickel, le ruthénium ou le fer.

2. Catalyseur selon la revendication 1 dans lequel dans le cas où la phase active est constituée de cobalt, nickel ou fer, les teneurs en métaux cobalt, nickel ou fer sont comprises entre 1 et 60 % poids, préférentiellement entre 5 et 30% poids, et dans le cas où la phase active est du ruthénium, la teneur en ruthénium est comprise entre 0,01 et 10% poids, préférentiellement entre 0,05 et 5% poids

3. Catalyseur selon l'une quelconque des revendications 1 à 2 dans lequel la silice alumine contient entre 1 et 30% poids de silice par rapport au produit anhydre et est homogène à l'échelle du micromètre, et de manière préférée, homogène à l'échelle du nanomètre.

4. Catalyseur selon l'une quelconque des revendications 1 à 3, dans lequel le métal du groupe VIII est le cobalt.

5. Catalyseur selon l'une quelconque des revendications 1 à 4 dans lequel la phase active contient au moins un élément additionnel choisi dans le groupe constitué par le ruthénium, le molybdène, le tantale, le platine, le palladium et le rhénium.

6. Catalyseur selon la revendication 5 dans lequel ledit élément additionnel est le ruthénium ou le rhénium.

7. Procédé de synthèse Fischer-Tropsch à partir d'un mélange de monoxyde de carbone et d'hydrogène, mettant en oeuvre le catalyseur selon l'une quelconque des revendications 1 à 6, dans lequel la mise en oeuvre est réalisée dans un réacteur triphasique et dans lequel le catalyseur est divisé à l'état de particules de diamètre compris entre 10 microns et 300 microns, préférentiellement compris entre 20 microns et 150 microns, et de manière encore plus préférée compris entre 20 et 120 microns.

## Patentansprüche

1. Katalysator, um die Synthese von Kohlenwasserstoffen aus einem Gemisch, umfassend Kohlenmonoxid und Wasserstoff, durchzuführen, umfassend:
- einen Träger, der von einer einfachen Spinell-Struktur des Typs MAl₂O₆/Al₂O₃.SiO₂ gebildet ist, wobei M Kobalt ist, und Al₂O₃.SiO₂ die chemische Formel eines Siliciumdioxids und Aluminiumoxids bezeichnet, wobei der Träger mindestens 10 Gew.-% einer Spinell-Struktur umfasst, wobei der Träger ein unter einer zumindest teilweise oxydierenden Atmosphäre bei einer Temperatur zwischen 850 °C und 900 °C kalzinierter Träger ist;
- eine Aktivphase, die auf den Träger aufgebracht ist, die ein oder mehrere Metalle der Gruppe VIII enthält, ausgewählt unter Kobalt, Nickel, Ruthenium und Eisen.

2. Katalysator nach Anspruch 1, bei dem, falls die Aktivphase von Kobalt, Nickel oder Eisen gebildet ist, der jeweilige Gehalt an Metallen Kobalt, Nickel oder Eisen zwischen 1 und 60 Gew.-%, vorzugsweise zwischen 5 und 30 Gew.-%, beträgt, und falls die Aktivphase Ruthenium ist, der Rutheniumgehalt zwischen 0,01 und 10 Gew.-%, vorzugsweise zwischen 0,05 und 5 Gew.-%, beträgt.

3. Katalysator nach einem der Ansprüche 1 bis 2, bei dem das Siliciumdioxid und Aluminiumoxid zwischen 1 und 30 Gew.-% Siliciumdioxid bezogen auf das wasserfreie Produkt enthält, und auf Mikrometerebene und bevorzugt auf Nanometerebene homogen ist.

4. Katalysator nach einem der Ansprüche 1 bis 3, bei dem das Metall der Gruppe VIII Kobalt ist.

5. Katalysator nach einem der Ansprüche 1 bis 4, bei dem die Aktivphase mindestens ein zusätzliches Element enthält, ausgewählt in der Gruppe, die von Ruthenium, Molybdän, Tantal, Platin, Palladium und Rhenium gebildet ist.

6. Katalysator nach Anspruch 5, bei dem das zusätzliche Element Ruthenium oder Rhenium ist.

7. Fischer-Tropsch-Syntheseverfahren aus einem Gemisch von Kohlenmonoxid und Wasserstoff, das den Katalysator nach einem der Ansprüche 1 bis 6 einsetzt, bei dem der Einsatz in einem Dreiphasenreaktor erfolgt, und bei dem der Katalysator in Form von Partikeln mit einem Durchmesser zwischen 10 Mikrometer und 300 Mikrometer, vorzugsweise zwischen 20 Mikrometer und 150 Mikrometer, und noch bevorzugter zwischen 20 und 120 Mikrometer, aufgeteilt ist.

## Claims

1. A catalyst intended for hydrocarbon synthesis from a mixture comprising carbon monoxide and hydrogen, comprising:
- a support consisting of a simple spinel structure of formula MAl₂O₄/Al₂O₃ SiO₂ wherein M is cobalt, and Al₂O₃ SiO₂ designating the chemical formula of a silica-alumina, said support comprises at least 10 wt % of said spinel structure, said support being a support calcined in an at least partly oxidizing atmosphere, at a temperature ranging between 850°C and 900°C,
- an active phase deposited on said support, which contains one or more group VIII metals, selected among cobalt, nickel, ruthenium or iron.

2. A catalyst as claimed in claim 1 wherein, in the case where the active phase consists of cobalt, nickel or iron, the proportions of metals cobalt, nickel or iron range between 1 and 60 wt %, preferably between 5 and 30 wt % and, if the active phase is ruthenium, the proportion of ruthenium ranges between 0.01 and 10 wt %, preferably between 0.05 and 5 wt %.

3. A catalyst as claimed in any one of claims 1 and 2, wherein the silica-alumina contains between 1 and 30 wt % of silica in relation to the anhydrous product, it is homogeneous on a micrometric scale and preferably on a nanometric scale.

4. A catalyst as claimed in any one of claims 1 to 3, wherein the group VIII metal is cobalt.

5. A catalyst as claimed in any one of claims 1 to 4, wherein the active phase contains at least one additional element selected from the group made up of ruthenium, molybdenum, tantalum, platinum, palladium and rhenium.

6. A catalyst as claimed in claim 5, wherein said additional element is ruthenium or rhenium.

7. A method intended for Fischer-Tropsch synthesis from a mixture of carbon monoxide and hydrogen, using the catalyst as claimed in any one of claims 1 to 6 in a three-phase reactor wherein the catalyst is divided into particles of diameter ranging between 10 microns and 300 microns, preferably between 20 microns and 150 microns, and more preferably between 20 microns and 120 microns.
